(19) Europäisches Patentamt
European Patent Office
Office européen des brevets

(11) Veröffentlichungsnummer: **0 018 532**
**A1**

(12) # EUROPÄISCHE PATENTANMELDUNG

(21) Anmeldenummer: 80101998.5

(22) Anmeldetag: 14.04.80

(51) Int. Cl.³: C 07 C 69/753
C 07 C 15/44, C 07 C 25/24
C 07 C 21/24, C 07 C 47/24

(30) Priorität: 23.04.79 DE 2916357

(43) Veröffentlichungstag der Anmeldung:
12.11.80 Patentblatt 80/23

(84) Benannte Vertragsstaaten:
BE CH DE FR GB IT LI NL

(71) Anmelder: **BAYER AG**
**ZENTRALBEREICH PATENTE, MARKEN UND LIZENZEN**
**D-5090 LEVERKUSEN 1, BAYERWERK(DE)**

(72) Erfinder: **Fuchs, Rainer, Dr.**
**Roeberstrasse 8**
**D-5600 Wuppertal(DE)**

(72) Erfinder: **Harnisch, Horst, Dr.**
**Heinenbusch 4**
**D-5203 Much(DE)**

(72) Erfinder: **Lantzsch, Reinhard, Dr.**
**Heymannstrasse 32**
**D-5090 Leverkusen 1(DE)**

(72) Erfinder: **Naumann, Klaus, Dr.**
**Richard-Wagner-Strasse 9**
**D-509 Leverkusen 1(DE)**

(72) Erfinder: **Riebel, Hans Jochem, Dr.**
**In der Beek 92**
**D-5600 Wuppertal 1(DE)**

(72) Erfinder: **Schröder, Rolf, Dr.**
**Pahlkestrasse 17**
**D-5600 Wuppertal 1(DE)**

(54) **Verfahren zur Herstellung von 3-(2-Aryl-vinyl)-2,2-dimethyl-cyclopropan-1-carbonsäureestern, neue Zwischenprodukte dafür und deren Herstellung.**

(57) Verfahren zur Herstellung von 3-(2-Aryl-vinyl)-2,2-dimethyl-cyclopropan -1-carbonsäureestern der Formel

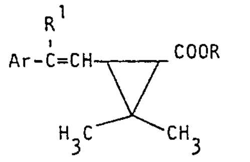

(1)

in welcher Ar für einen carbocyclischen oder heterocyclischen aromatischen Rest, R für Alkyl sowie für einen bei Pyrethroiden üblichen Rest und R' für Wasserstoff oder Halogen stehen, durch Umsetzung entsprechender 1-Aryl-4-methylpenta-1, 3-diene (II) mit entsprechenden Diazoessigsäureestern, neue Ausgangsverbindungen II selbst und mehrere verschiedene Verfahren zur Herstellung derselben, beispielsweise durch Umsetzung eines $\beta$-Aryl-acroleins der Formel

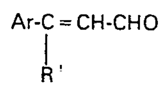

(VII)

worin Ar und R' obige Bedeutung haben mit Triphenylisopropylphosphoran oder mit Isopropylmagesiumchlorid sowie ein Verfahren zur Herstellung der Verbindung VII in der R' für Halogen steht durch Umsetzung eines Arylmethylketons mit Dimethylformamid und Phosphoroxyhalogenid. Die Verbindungen der Formel I können als Zwischenprodukte zur Herstellung von Insektiziden verwendet werden.

EP 0 018 532 A1

Croydon Printing Company Ltd.

BEZEICHNUNG GEÄNDERT
siehe Titelseite

BAYER AKTIENGESELLSCHAFT
5090 Leverkusen, Bayerwerk
Zentralbereich
Patente, Marken und Lizenzen
Rt/Kü
IVa/ZP

Verfahren zur Herstellung von 3-(2-Aryl-vinyl)-2,2-dimethyl-cyclopropan-1-carbonsäureestern und neue Zwischenprodukte dafür

Die Erfindung betrifft ein neues Verfahren zur Herstellung von teilweise bekannten 3-(2-Aryl-vinyl)-2,2-dimethyl-cyclopropan-1-carbonsäureestern, und neue Zwischenprodukte zur Durchführung dieses Verfahrens sowie deren Herstellung.

Es ist bekannt, daß man bestimmte 3-(2-Chloro-2-aryl-vinyl)-2,2-dimethyl-cyclopropan-1-carbonsäureester, wie z.B. 3-(2-Chloro-2-phenyl-vinyl)-2,2-dimethyl-cyclopropan-1-carbonsäure-äthylester erhält, wenn man entsprechende Arylmethan-phosphonsäureester mit einer starken Base, wie z.B. Butyllithium, behandelt und dann nacheinander Tetrachlorkohlenstoff und 3-Formyl-2,2-dimethyl-cyclopropan-1-carbonsäureester dazu gibt (vergleiche DE-OS 2 738 15o).

Dieses Herstellungsverfahren für 3-(2-Chloro-2-aryl-vinyl)-2,2-dimethyl-cyclopropan-1-carbonsäureester weist jedoch eine Reihe von Nachteilen auf:



BAD ORIGINAL

Die zur Deprotonierung der Arylmethanphosphonsäureester benötigten starken Basen, wie z.B. Butyllithium, sind feuchtigkeits- und luftempfindlich; daher wird das konventionelle Verfahren in einer Inertgasatomsphäre, beispielsweise unter Stickstoff oder Argon, und unter Verwendung eines inerten, sorgfältig getrockneten Lösungs- oder Verdünnungsmittels durchgeführt. Da die Umsetzung bei tiefer Temperatur, etwa bei -7o°C durchzuführen ist, muß das Reaktionsgemisch auch intensiv gekühlt werden. Die Aufarbeitung des Reaktionsgemisches zur Isolierung der gewünschten Produkte, welche man als Isomerengemische erhält, ist ebenfalls aufwendig; sie umfaßt mehrere Extraktionen und Lösungsmitteldestillationen sowie einen chromatographischen Trennungsprozeß. Die oben erläuterte bekannte Synthesemethode dürfte daher für eine Herstellung der 3-(2-Chloro-2-aryl-vinyl)-2,2-dimethyl-cyclopropan-1-carbonsäureester im industriellen Maßstab wenig geeignet sein und sollte möglichst durch ein einfacher durchführbares Verfahren ersetzt werden.

Gegenstand der vorliegenden Erfindung sind

(1) ein Verfahren zur Herstellung von 3-(2-Aryl-vinyl)-2,2-dimethyl-cyclopropan-1-carbonsäureestern der Formel I

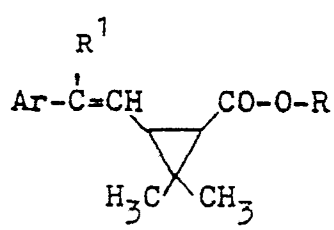

(I)



BAD ORIGINAL

in welcher

Ar für substituierte oder unsubstituierte carbocyclische oder heterocyclische aromatische Reste steht und

R für Alkyl sowie für Reste der bei Pyrethroiden üblichen Alkohole steht, und

$R^1$ für Wasserstoff oder Halogen steht,

dadurch gekennzeichent, daß man 1-Aryl-4-methyl-penta-1,3-diene der Formel II

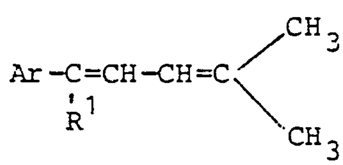

(II)

in welcher

Ar und $R^1$ die oben angegebene Bedeutung hat,

mit Diazoessigsäureestern der Formel III

$$N_2CH\text{-}COO\text{-}R \qquad (III)$$

in welcher

R die oben angegebene Bedeutung hat,

in Gegenwart eines Katalysators bei Temperaturen zwischen 5o und 2oo°C umsetzt;



BAD ORIGINAL

(2) neue 1-Aryl-4-methyl-penta-1,3-diene der Formel (II) (oben), worin Ar und $R^1$ die oben angegebene Bedeutung haben;

(3) ein Verfahren zur Herstellung von 1-Halogen-1-aryl-4-methyl-penta-1,3-dienen der Formel II (oben), worin Ar die oben angegebene Bedeutung hat, und $R^1$ für Halogen steht, das dadurch gekennzeichnet ist, daß man 3-Methyl-buten-2-yl-arylketone der Formel IV a

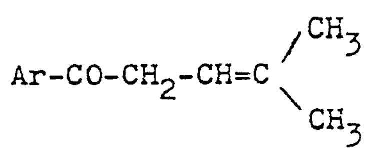

(IV a)

und/oder die dazu isomeren 3-Methyl-buten-1-yl-arylketone der Formel IV b

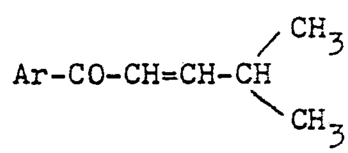

(IV b)

worin Ar die oben angegebene Bedeutung hat,

mit Phosphorpentahalogenid, gegebenenfalls unter Verwendung eines inerten Verdünnungsmittels, bei Temperaturen zwischen -4o°C und +3o°C umsetzt und anschließend das Reaktionsgemisch im gleichen Temperaturbereich mit eine Säurebindemittel, gegebenenfalls in Gegenwart eines Katalysators, behandelt;

(4) ein Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man Arylmethan-phosphonsäureester der Formel V

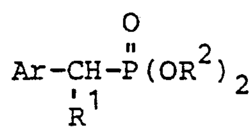

(V)

in welcher

Ar und $R^1$ die oben angegebene Bedeutung hat und

$R^2$ für Alkyl oder Phenyl steht oder worin die beiden Reste $R^2$ zusammen für geradkettiges oder verzweigtes Alkandiyl stehen,

mit Dimethylacrolein der Formel VI

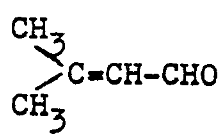

(VI)

in Gegenwart einer Base und gegebenenfalls unter Verwendung eines Verdünnungsmittels bei Temperaturen zwischen -7o und +15o°C umsetzt;

(5) ein Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man ß-Aryl-acroleine der Formel VII

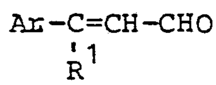 (VII)

in welcher

Ar und $R^1$ die oben angegebene Bedeutung haben,

mit Triphenyl-isopropyl-phosphoran der Formel VIII

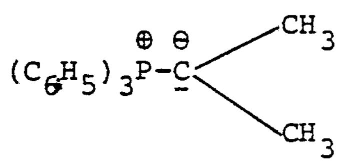 (VIII)

gegebenenfalls unter Verwendung von Verdünnungsmitteln bei Temperaturen zwischen -70 und +50°C umsetzt;

(6) ein Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man ß-Aryl-acroleine der Formel VII (oben), worin Ar und $R^1$ die oben angegebene Bedeutung haben, mit Isopropylmagnesiumchlorid der Formel IX

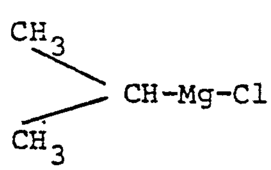 (IX)

gegebenenfalls in Gegenwart von Verdünnungsmitteln bei Temperaturen zwischen -7o und +5o°C umsetzt und anschließend die dabei gebildeten Produkte durch Behandeln mit Säuren bei 0 bis 5o°C dehydratisiert;

(7) ein Verfahren zur Herstellung von ß-Halogen-ß-Arylacroleinen der Formel VII (oben), worin Ar die oben angegebene Bedeutung hat, und $R^1$ für Halogen steht, dadurch gekennzeichnet, daß man Aryl-methyl-ketone der Formel X

$$Ar-CO-CH_3 \qquad (X)$$

in welcher Ar die oben angegebene Bedeutung hat,

mit Dimethylformamid und Phosphoroxyhalogenid bei Temperaturen zwischen 0 und 100°C ohne Zusatz eines weiteren Verdünnungsmittels umsetzt und die Reaktionsprodukte ohne destillative Aufarbeitung isoliert.

Überraschenderweise können nach dem neuen Verfahren, welches mit geringerem Aufwand als das bekannte Verfahren durchzuführen ist und bei dem einfach herzustellende Ausgangsverbindungen eingesetzt werden können, 3-(2-Aryl-vinyl)-2,2-dimethyl-cyclopropan-1-carbonsäureester in guten Ausbeuten hergestellt werden.

Nach dem erfindungsgemäßen Verfahren (1) werden vorzugsweise 3-( 2-Aryl-vinyl)-2,2-dimethyl-cyclopropan-1-carbonsäureester der Formel (I) hergestellt,

in welcher

Ar für einen gegebenenfalls durch einen oder mehrere gleiche oder verschiedene Substituenten X substituierten Phenyl- oder Naphthyl-Rest steht, wobei

X für Halogen, Alkyl, Cyano, Nitro, Aryl, Aralkyl, Alkoxy, Aryloxy, Arylthio, Alkylthio, Halogenalkyl, Halogenalkoxy, Halogenalkylthio gegebenenfalls halogensubstituiertes Methylendioxy oder Äthylendioxy steht und

$R^1$ für Chlor oder Brom steht.

Besonders bevorzugt werden Verbindungen der Formel I hergestellt,

in welcher

Ar für einen gegebenenfalls durch einen oder mehrere gleiche oder verschiedene Substituenten X substituierten Phenylrest steht, wobei

X für Fluor, Chlor, Brom, Cyano, Nitro, $C_1$-$C_4$-Alkyl, $C_1$-$C_2$-Alkoxy, $C_1$-$C_2$-Alkylthio, Trifluormethyl, $C_1$-$C_2$-Fluoralkoxy oder $C_1$-$C_2$-Chlorfluoralkoxy, Trifluormethylthio, Methylendioxy, Difluormethylendioxy, Äthylendioxy, Difluoro-, Trifluoro- oder Tetrafluoro-äthylendioxy steht und

$R^1$ für Chlor steht, und

R für $C_1$-$C_4$-Alkyl steht.

Besonders bevorzugte, nach dem erfindungsgemäßen Verfahren (1) herstellbare Verbindungen sind 3-(2-Chloro-2-aryl-vinyl)-2,2-dimethyl-cyclopropan-1-carbonsäureester der Formel (I)

in welcher

Ar für einen gegebenenfalls durch einen oder zwei gleiche oder verschiedene Substituenten X in 3- oder 4-Stellung substituierten Phenylrest steht, wobei

X für Fluor, Chlor, Brom, Methyl, Methoxy, Methylthio, Trifluormethyl, Trifluormethoxy, Trifluormethylthio, Methylendioxy, Difluormethylendioxy oder Trifluoräthylendioxy steht und

$R^1$ für Chlor steht, und

R für Methyl oder Äthyl steht.

Die allgemeine Formel (I) umfaßt die verschiedenen Isomeren und deren Mischungen. Die Verbindungen der Formel I sind zum Teil bekannte Insektizide zum Teil Zwischenprodukte zur Herstellung von Insektiziden.

Verwendet man beim erfindungsgemäßen Verfahren (1) als Ausgangsstoffe beispielsweise 1-Chloro-1-phenyl-4-methyl-pentadien und Diazoessigsäureäthylester, so kann die Reaktion dieser Verbindungen durch folgendes Formelschema skizziert werden:

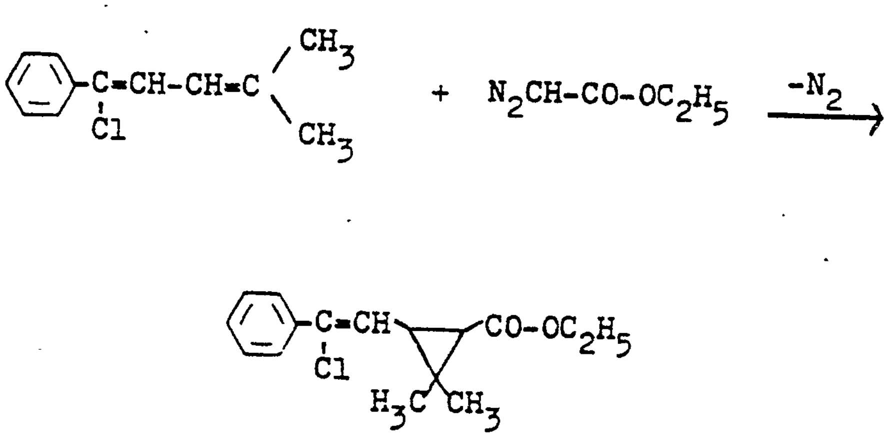


BAD ORIGINAL

Die als Ausgangsstoffe für Verfahren (I) zu verwendenden Verbindungen sind durch die Formeln (II) und (III) definiert. Vorzugsweise stehen darin Ar, $R^1$ und R für diejenigen Reste, welche bei der Definition der Reste Ar, $R^1$ und R in Formel (I) als bevorzugt genannt wurden. Die Diazoessigsäureester der Formel (III) sind literaturbekannte Verbindungen oder können analog bekannter Verfahren hergestellt werden. Als Beispiele seien genannt: Diazoessigsäuremethylester und -äthylester.

Die neuen 1-Aryl-4-methyl-penta-1,3-diene der Formel (II) können nach den unter (3), (4), (5) und (6) angegebenen Verfahren hergestellt werden. Als Beispiele für die Verbindungen der Formel (II) seien genannt:

1-Phenyl-, 1-(4-Fluoro-phenyl)-, 1-(4-Chloro-phenyl)-, 1-(4-Bromo-phenyl)-, 1-(3-Bromo-phenyl)-, 1-(3,4-Dichloro-phenyl)-, 1-(4-Methyl-phenyl)-, 1-(3-Trifluoromethyl-phenyl)- und 1-(4-Trifluormethyl-phenyl)-1-chloro-4-methyl-penta-1,3-dien.

Verwendet man bei Verfahren (3) als Ausgangsstoffe beispielsweise ein Gemisch aus 2-Methyl-5-oxo-5-(4-chloro-phenyl)-2-penten und 2-Methyl-5-oxo-5-(4-chloro-phenyl)-3-penten, so kann die Reaktion dieser Verbindungen mit Phosphorpentachlorid, welcher eine Chlorwasserstoff-Eliminierung folgt, durch nachstehendes Formelschema skizziert werden:

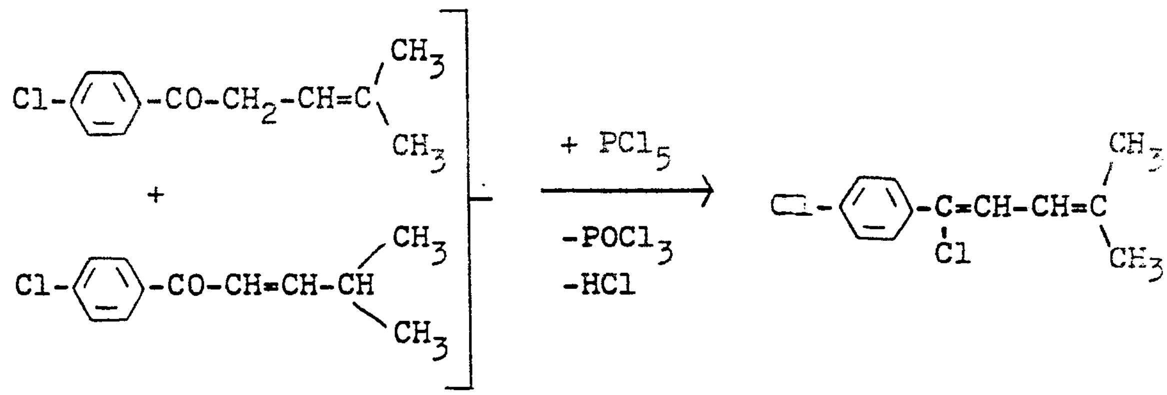


Die bei Verfahren (3) zu verwendenden Ausgangsstoffe sind durch die Formeln (IV a) und (IV b) definiert. Die bevorzugten und besonders bevorzugten Reste Ar sind die gleichen, wie bei den nach Verfahren (1) herzustellenden Verbindungen der Formel (I).

Die Ausgangsverbindungen der Formeln (IV a) und (IV b) sind teilweise bekannt und/oder können nach einem bekannten Verfahren hergestellt werden (vergleiche J.Org.Chem. 25 (196o), 272). Man erhält die Verbindungen der Formeln (IV a) und (IV b) beispielsweise, indem man Arylmethylketone der Formel X (oben) mit Isobutyraldehyd umsetzt und die Umsetzungsprodukte in Gegenwart von Natriumacetat destilliert.
Als Beispiele für die Aryl-methylketone der Formel X seien genannt:
Acetophenon, 4-Fluoro-acetophenon, 4-Chloro-acetophenon, 4-Bromo-acetophenon und 4-Methyl-acetophenon.



BAD ORIGINAL

Zur Durchführung von Verfahren (3) werden die Ausgangsstoffe der Formel (IV a) bzw. (IV b) bzw. deren Gemische in einem Verdünnungsmittel vorgelegt und mit Phosphorpentachlorid versetzt. Im Gegensatz zur allgemein üblichen Arbeitsweise bei der Umsetzung von Ketonen mit Phosphorpentachlorid (vergleiche Houben-Weyl, Methoden der organischen Chemie, Band 5/3, S. 912) wird Verfahren (3) vorzugsweise in Gegenwart eines Verdünnungsmittels durchgeführt. Als Verdünnungsmittel kommen beispielsweise Kohlenwasserstoffe, wie Benzin, Petroläther, Pentan, Hexan, Cyclohexan, Benzol, Toluol, Xylol oder Chlorkohlenwasserstoffe, wie Methylenchlorid, Tetrachlorkohlenstoff, Dichloräthan oder Chlorbenzol sowie Nitrile wie Acetonitril in Frage. Vorzugsweise werden Toluol und/oder Cyclohexan als Verdünnungsmittel verwendet. Die Umsetzung ungesättigter Ketone mit Phosphorpentachlorid ist praktisch immer problematisch und gibt Anlaß zu einer Vielzahl von Nebenreaktionen. So kann beispielsweise eine Chlorierung in Allylstellung bzw. in $\alpha$-Stellung zur Carbonylgruppe erfolgen. Der hierbei wie auch bei der Eliminierung entstehende Chlorwasserstoff kann sich wieder an eine Doppelbindung addieren. Außerdem können auch Doppelbindungen durch Phosphorpentachlorid chloriert werden.

Es wurde gefunden, daß sich diese Nebenreaktionen praktisch vollständig vermeiden lassen, wenn man in Gegenwart von Verdünnungsmitteln und bei relativ tiefer Temperatur arbeitet und anschließend bei der gleichen oder bei einer niedrigeren Temperatur eine Base zusetzt, sodaß das Vorhandensein von Chlorwasserstoff bei höheren Temperaturen vermieden wird. Das Verfahren wird im allgemeinen bei Temperaturen zwischen -4o und +3o°C, vorzugsweise zwischen -lo und +2o°C durchgeführt.

Als Säurebindemittel können bei Verfahren (3) praktisch alle technisch gebräuchlichen Basen verwendet werden. Hierzu gehören Hydroxide wie z.B. Natrium- und Kaliumhydroxid, Carbonate wie z.B. Natrium- und Kaliumcarbonat sowie Alkoholate wie z.B. Natriummethylat und -äthylat. Vorzugsweise werden wässrige Lösungen von Alkalihydroxiden oder -carbonaten in Gegenwart von Phasentransferkatalysatoren eingesetzt. Es können die üblichen Phasentransferkatalysatoren verwendet werden; vorzugsweise werden Tetraalkyl- oder Aralkyl-trialkylammoniumsalze wie z.B. Benzyl-triäthylammoniumchlorid oder Tetrabutylammoniumbromid eingesetzt.

Die nach Verfahren (3) hergestellten 1-Chloro-1-aryl-4-methyl-penta-1,3-diene können im allgemeinen durch Destillation gereinigt werden. Soweit diese Diene nicht unzersetzt destillierbar sind, erfolgt die Reinigung durch sogenanntes "Andestillieren", d.h. durch längeres Erhitzen auf mäßig erhöhte Temperaturen unter vermindertem Druck.

Verwendet man bei Verfahren (4) als Ausgangsstoffe beispielsweise 4-Trifluoromethyl-phenylbenzyl-phosphonsäurediäthylester und Dimethylacrolein, so kann die Reaktion dieser Verbindungen durch folgendes Formelschema skizziert werden:



BAD ORIGINAL

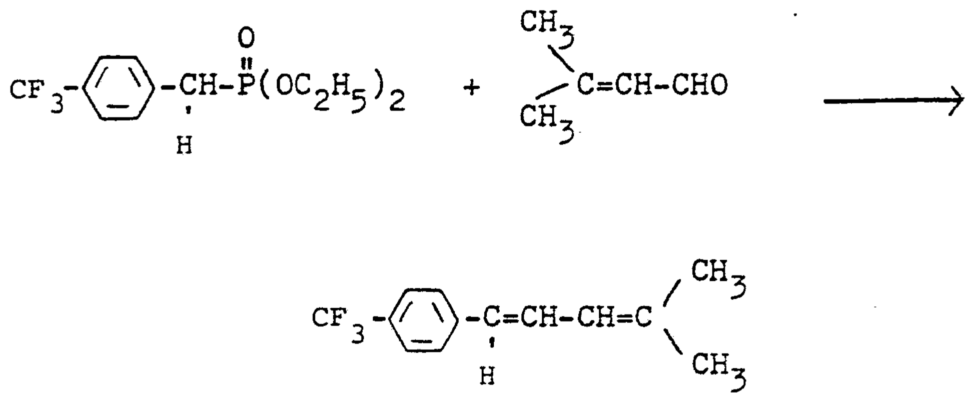

Die bei Verfahren (4) zu verwendenden Ausgangsstoffe sind durch die Formeln (V) und (VI) definiert. Die bevorzugten Reste Ar, $R^1$ und R sind die gleiche wie bei den nach Verfahren (1) herzustellenden Verbindungen der Formel (I). Dimethylacrolein (VI) ist eine literaturbekannte Verbindung.

Die Arylmethan-phosphonsäureester der Formel (V) sind teilweise bekannt. Verbindungen, in denen $R^1$ für Halogen steht können durch Umsetzung von $\alpha$-Hydroxy-arylmethanphosphonsäureestern der Formel XI

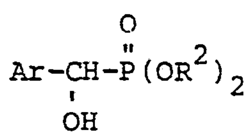

(XI)

in welcher Ar und $R^2$ die oben angegebene Bedeutung haben,

mit einem Halogenierungsmittel wie z.B. Thionylchlorid bei Temperaturen zwischen 0 und 100°C, vorzugsweise bei 20 bis 80°C, hergestellt werden (vergleiche Chimia 28 (1974), 656-657; J.Am.Chem.Soc. 87 (1965), 2777-2778).

Die α-Hydroxy-arylmethan-phosphonsäureester der Formel (XI) sind teilweise bekannt. Man erhält sie nach bekannten Verfahren, im allgemeinen durch Umsetzung von Aldehyden der Formel XII

$$Ar\text{-}CHO \qquad (XII)$$

in welcher Ar die oben angegebene Bedeutung hat,

mit Phosphorigsäureestern der Formel XIII

$$H\text{-}\overset{O}{\overset{\|}{P}}(OR^1)_2 \qquad (XIII)$$

in welcher $R^1$ die oben angegebene Bedeutung hat, gegebenenfalls in Gegenwart eines Katalysators wie z.B. Triäthylamin bei Temperaturen zwischen 0 und 15o°C, vorzugsweise bei 2o bis loo°C (vergleiche Houben-Weyl, Methode der organischen Chemie, 4. Aufl. (1963), Band <u>12/1</u>, S. 475-483).

Als Beispiele für die Aldehyde der Formel (XII) seien genannt:
Benzaldehyd, 4-Chlorobenzaldehyd, 4-Fluoro-benzaldehyd, 3-Bromo-benzaldehyd, 4-Bromo-benzaldehyd, 3-Chloro-benzaldehyd. 3.4-Dichloro-benzaldehyd, 2,6-Difluoro-benzaldehyd und 4-Methyl-benzaldehyd.

Als Beispiele für die Phosphorigsäureester der Formel (XIII) seien Phosphorigsäuredimethylester und Phosphorigsäurediäthylester genannt:

Verfahren (4) ist eine Variante der Wittig-Horner-Reaktion. Aus der Literatur sind für diese Variante nur wenige Beispiele bekannt. Als Base wird nach dem Stand der Technik Natriumhydrid verwendet, welches ein teures und schwierig zu handhabendes Reagenz ist. Dagegen kann man nach dem neuen Verfahren wesentlich einfacher und kostengünstiger arbeiten. Man kann die Umsetzung bei Raumtemperatur in wasserhaltigen Reaktionsmedien durchführen und an Stelle von Natriumhydrid billigere Basen verwenden.

Verfahren (4) wird vorzugsweise unter Verwendung von Verdünnungsmitteln durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan. Alkohole wie Methanol, Äthanol, n- und iso-Propanol, n-, iso-, sek.- und tert.-Butanol sowie Dimethylsulfoxid.

Bei Arbeiten im Zweiphasen-medium werden neben 5o%iger wässriger Natronlauge mit Wasser praktisch nicht mischbare Solventien wie z.B. Benzin, Benzol oder Toluol verwendet.

Als Basen können die bei Carbonyl-Olefinierungen üblichen Basen verwendet werden. Genannt seien Alkalihydroxide wie z.B. Natrium- und Kaliumhydroxid, Alkalialkoholate wie z.B. Natrium- und Kaliummethylat, -äthylat, -n- und -iso-propylat, -n-, -iso-, -sek.- und tert.-butylat, Alkalihydride wie z.B. Natriumhydrid sowie Alkyllithiumverbindungen wie z.B. Butyllithium. Vorzugsweise werden als Basen Natrium- und Kaliumhydroxid sowie Natriummethylat und -äthylat verwendet.

Die Reaktionstemperaturen liegen im allgemeinen zwischen -7o und +15o°C, vorzugsweise zwischen -1o und + 5o°C. Die Umsetzung wird im allgemeinen bei Normaldruck durchgeführt. Zur Durchführung von Verfahren (4) werden die Komponenten gewöhnlich in äquimolaren Mengen eingesetzt. Lediglich die Basen werden gewöhnlich in größerem Überschuß eingesetzt; bei Arbeiten im einphasigen System bis zu 3o Mol-%, vorzugsweise bis zu 15 mol-%; bei Verwendung von 5o%iger Natronlauge als zweiter Phase im allgemeinen das 5 bis 15fache der stöchiometrisch erforderlichen Menge.

Alkoholate werden gegebenenfalls in situ aus den Alkoholen und Alkalimetallen hergestellt.

BAD ORIGINAL

Im allgemeinen wird die Base und gegebenenfalls der Katalysator in einem oder mehreren der genannten Verdünnungsmittel vorgelegt. Dazu werden, gegebenenfalls in einem der angegebenen Solventien gelöst, die Reaktionskomponenten - Arylmethan-phosphonsäureester (V) und Dimethylacrolein (VI) - in der angegebenen Reihenfolge zugegeben. Zur Vervollständigung der Reaktion wird die Mischung unter Rühren mehrere Stunden im angegebenen Temperaturbereich gehalten. Zur Aufarbeitung versetzt man die Reaktionsmischung mit Wasser, säuert gegebenenfalls mit Salzsäure an und extrahiert mit Methylenchlorid. Die organische Phase wird getrocknet und das Lösungsmittel wird im Vakuum andestilliert. Das als Rückstand verbleibende Produkt wird gegebenenfalls durch Vakuumdestillation, oder falls es nicht unzersetzt destillierbar ist, durch sogenanntes "Andestillieren", d.h. durch längeres Erhitzen unter vermindertem Druck auf mäßig erhöhte Temperaturen, gereinigt. Zur Charakterisierung dient der Siedepunkt oder der Brechungsindex.

Verwendet man bei Verfahren (5) als Ausgangsstoffe ß-Chloro-ß-(3-methyl-phenyl)-acrolein und Isopropylphosphoran, so kann die Reaktion dieser Verbindungen durch folgendes Formelschema skizziert werden:

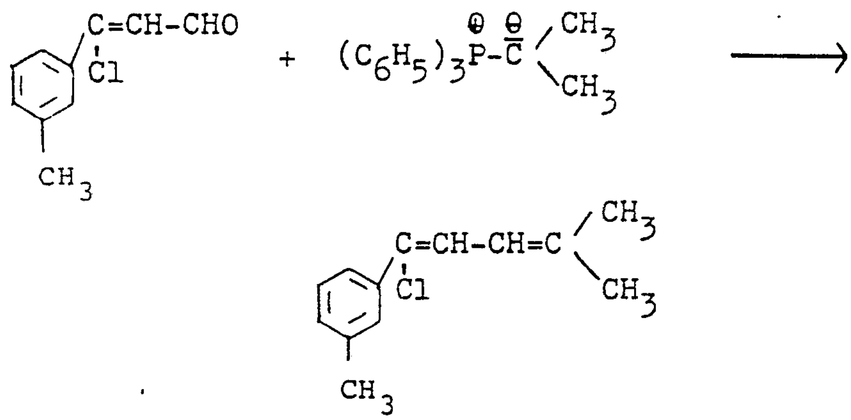

Die Herstellung des Phosphorans (VIII) ist bekannt.

Die Acroleinderivate der Formel (VII) sind teilweise bekannt. Ein neues Verfahren (7) zu ihrer Herstellung wird weiter unten beschrieben.

Die bei Verfahren (5) zu verwendenden Ausgangsstoffe sind durch die Formeln (VII) und (VIII) definiert. Die bevorzugten und besonders bevorzugten Reste und Substituenten sind die gleichen wie bei den nach Verfahren (1) herzustellenden Verbindungen der Formel (I). Als Beispiele für die Acroleinderivate der Formel (VII) seien genannt:
ß-Phenyl-, ß-(4-Fluoro-phenyl)-, ß-(4-Chloro-phenyl)-, ß-(4-Bromo-phenyl)- und ß-(4-Methyl -phenyl)- acrolein sowie die entsprechenden ß-Chloro-acroleine.

Verfahren (5) wird nach den bei Carbonyl-Olefinierungen nach Wittig üblichen Methoden durchgeführt.

Verwendet man bei Verfahren (6) als Ausgangsstoffe ß-Chloro-ß-(4-methyl -phenyl)-acrolein und Isopropylmagnesiumchlorid, so kann die Reaktion dieser Verbindungen durch folgendes Formelschema skizziert werden:

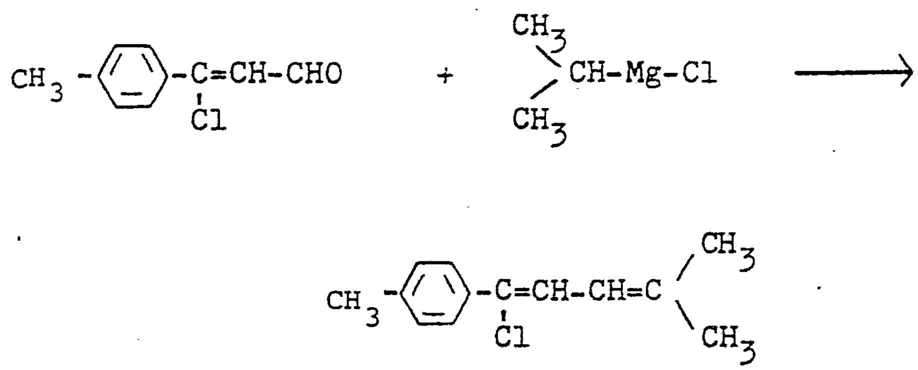

Isopropylmagnesiumchlorid kann nach einem bekannten Verfahren hergestellt werden (vergleiche Chem.Ber.69 (1936), 1766).

Die bei Verfahren (6) zu verwendenden Ausgangsstoffe sind durch die Formeln (VII) und (IX) definiert. Die bevorzugten und besonders bevorzugten Reste und Substituenten sind die gleichen wie bei den nach Verfahren (1) herzustellenden Verbindungen der Formel (I). Als Beispiele für die Acroleinderivate der Formel (VII) seien genannt:
ß-Phenyl-, ß-(4-Fluoro-phenyl)-, ß-(4-Chloro-phenyl)-, ß-(4-Bromo-phenyl)- und ß-(4-Methyl-phenyl)- acrolein, sowie die entsprechenden ß-Chloro-acroleine.

Verfahren (6) ist eine Grignard-Reaktion mit nachfolgender Dehydratisierung. Die Grignard-Reaktion kann auf bekannte Weise durchgeführt werden. Es ist als überraschend anzusehen, daß aus dem intermediär gebildeten sekundären Alkohol verhältnismäßig leicht Wasser eliminiert werden kann. Die Dehydratisierung erfolgt mit Säuren, vorzugsweise mit Schwefelsäure bei Temperaturen zwischen 0 und 5o°C, vorzugsweise bei 2o bis 45°C. Die Aufarbeitung erfolgt nach Eingießen in Wasser durch Extraktion nach üblichen Methoden.

Verwendet man bei Verfahren (7) als Ausgangsstoffe beispielsweise 4-Fluoro-acetophenon, Dimethylformamid und Phosphoroxychlorid, so kann die Reaktion durch folgendes Formelschema skizziert werden:

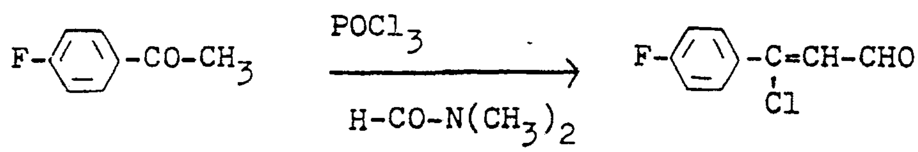


Die bei Verfahren (7) als Ausgangsstoffe zu verwendenden Aryl-methylketone sind durch Formel (X) definiert. Die bevorzugten und besonders bevorzugten Reste und Substituenten sind die gleichen wie bei den nach Verfahren (7) herzustellenden Verbindungen der Formel (I). Als Beispiele für die Aryl-methylketone der Formel (X) seien genannt:
Acetophenon, 4-Fluoroacetophenon, 4-Chloroacetophenon, 4-Bromoacetophenon, 4-Methylacetophenon und



BAD ORIGINAL

4-Phenyl-acetophenon.

Man erhält nach Verfahren (7) ß-Halogen-ß-aryl-acroleine der Formel (VII) in besonders einfacher Weise und mit verbesserter Ausbeute und Qualität, wenn man auf den in der Literatur (Proc.Chem.Soc. (London) 1958, 227; Angew.Chem. 71 (1959), 573; Chem.Ber. 93 (196o), 2746 und Chem.Ber. 98 (1965), 3554) empfohlenen Zusatz eines Verdünnungsmittels und die verlustreiche destillative Aufarbeitung verzichtet und die Produkte nach schonender Hydrolyse bei tiefer Temperatur in kristalliner Form isoliert. Man geht bei Verfahren (7) so vor, daß man das Keton der Formel (X) in Dimethylformamid löst und bei Temperaturen zwischen 0 und 1oo°C, vorzugsweise zwischen 2o und 6o°C mit Phosphoroxychlorid versetzt und bei der gleichen Temperatur 1 bis 5 Stunden nachrührt. Anschließend wird auf Eiswasser gegeben und mit Natronlauge ein pH-Wert von ungefähr 5 eingestellt. Die Temperatur soll dabei nicht über 25°C steigen. Nach einigem Nachrühren wird abgesaugt, mit Wasser gewaschen und im Vakuum getrocknet.

Das erfindungsgemäße Verfahren (1) wird in Gegenwart von Katalysatoren durchgeführt. Als solche kommen Kupfer und Kupferverbindungen verschiedener Oxidationsstufen - auch Gemische - in Frage, wie z.B. Kupfer (als Pulver oder Bronze), Kupfer(I)- und -(II)chlorid, Kupfer(I)- und -(II)-oxid sowie Kupfer(II)sulfat. Die Reaktionstemperatur kann innerhalb eines größeren Bereichs variiert werden. Im allgemeinen arbeitet man zwischen 5o und 2oo°C, vorzugsweise bei 8o bis 15o°C.

Das erfindungsgemäße Verfahren wird im allgemeinen bei Normaldruck durchgeführt. Bei der Durchführung von Verfahren (1) werden die 1-Aryl-4-methyl-penta-1,3-diene (II) und die Diazoessigsäureester (III) im Molverhältnis 1:1 bis 20:1 eingesetzt. Im allgemeinen werden etwa $^2/_3$ der einzusetzenden Menge an 1-Aryl-4-methyl-penta-1,3-dien (II) zusammen mit dem Katalysator auf die erforderliche Reaktionstemperatur erhitzt und dann tropfenweise mit dem Diazoessigsäureester (III), welcher mit dem restlichen Drittel des Diens (II) verdünnt ist, versetzt. Nach Ende der Stickstoffentwicklung wird das Reaktionsgemisch abgekühlt, mit einem mit Wasser nicht mischbaren Lösungsmittel, wie z.B. Methylenchlorid, verdünnt und filtriert. Das Filtrat wird mit Wasser gewaschen, getrocknet und destillativ aufgearbeitet. Zur Charakterisierung der Produkte dienen die Siedepunkte.

Nach dem erfindungsgemäßen Verfahren herstellbare Verbindungen der Formel (I) können als Zwischenprodukte zur Herstellung von Insektiziden verwendet werden (vergleiche DE-OS 2 738 150).



BAD ORIGINAL

Herstellungsbeispiele

Beispiele zu Verfahren (1):

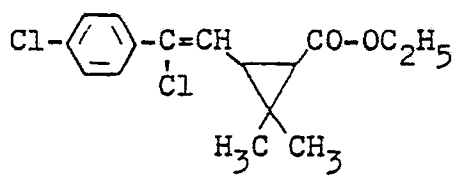

Eine Mischung von 45,4 g (o,2 Mol) 1-(4-Chloro-phenyl)-1-chloro-4-methyl-penta-1,3-dien, 1,2 g Kupferpulver und 1,5 g Kupfersulfat (wasserfrei) wird auf 11o°C erhitzt und dann unter Rühren ebenfalls bei 11o°C eine Mischung von 22,7 g (o,1 Mol) 1-(4-Chloro-phenyl)-1-chloro-4-methyl-penta-1,3-dien und 34,2 g (o,3 Mol) Diazoessigsäureäthylester, sehr langsam, innerhalb von 5 Stunden, zugetropft. Nach beendeter Stickstoffentwicklung wird die Mischung abgekühlt, mit 5oo ml Methylenchlorid verdünnt und dann filtriert. Das Filtrat wird mit 5oo ml Wasser ausgeschüttelt, anschließend wird die organische Phase abgetrennt, über Magnesiumsulfat getrocknet und dann das Lösungsmittel im Wasserstrahlvakuum abdestilliert. Der ölige Rückstand wird im Vakuum destilliert. Man erhält dabei zwei Fraktionen:

Fraktion 1: Siedepunkt 124-135°C/2 Torr

Fraktion 2: Siedepunkt 135-17o°C/2 Torr

Fraktion 1 erweist sich als nicht umgesetztes 1-(4-Chlorophenyl)-1-chloro-4-methyl-penta-1,3-dien. Fraktion 2 wird nochmals destilliert. Man erhält dann 11,2 g 2,2-Dimethyl-3-(2-chloro-2-(4-chloro-phenyl)-vinyl)-cyclopropan-carbonsäureäthylester (Isomerengemisch) als gelbes Öl mit dem Siedepunkt 146-17o°C/2 Torr.

Analog erhält man:

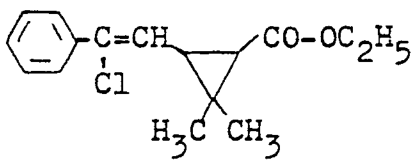

Siedepunkt:
14o-15o°C/3mbar

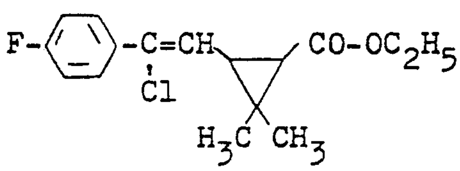

Siedepunkt:
135-145°C/3mbar

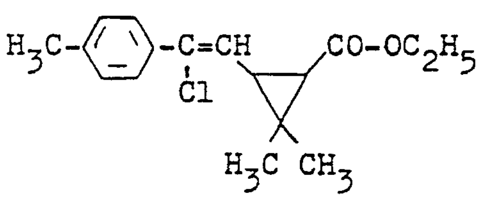

Siedepunkt:
16o-175°C/4mbar

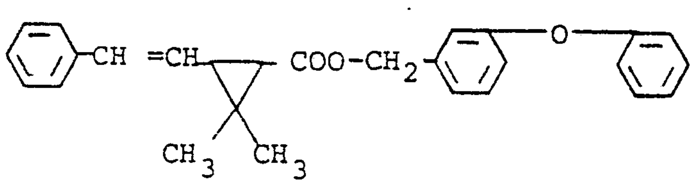

Beispiel zu Verfahren 3:

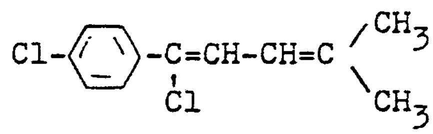

Man löst 17 g Kaliumhydroxid in 125 ml Wasser und 125 ml Methanol und gibt 155 g 4-Chloracetophenon hinzu. Bei 5o°C werden 8o g Isobutyraldehyd innerhalb von 1,5 Stunden zugetropft. Nach 3,5 Stunden wird auf Raumtemperatur abgekühlt und mit ca. 2o ml Essigsäure neutralisiert. Das dimere Kondensationsprodukt wird abgesaugt, mit Methanol gewaschen und an der Luft getrocknet. Ausbeute 185 g (88,5 % der Theorie), Schmelzpunkt 14o°C. Der Feststoff wird mit 5 g Natriumacetat vermischt und im Wasserstrahlvakuum destilliert. Man erhält 175 g eines bei Raumtemperatur erstarrenden gelben Öls, das ein Gemisch aus 2-Methyl-5-oxo-5-(4-chloro-phenyl)-2-penten und 2-Methyl-5-oxo-5-(4-chloro -phenyl)-3-penten darstellt. Das erstere ist ein Feststoff und kristallisiert bevorzugt aus. Siedepunkt des Isomerengemisches: 155-157°C/18 mbar.

Man löst 1o4,25 g (o,5 Mol) des oben erhaltenen Isomerengemisches in 5oo ml Toluol und dosiert 1o4 g Phosphorpentachlorid bei 0°C langsam hinzu. Man rührt solange bei 0-1o°C, bis alles $PCl_5$ in Lösung gegangen ist, gibt 5 g Tetrabutylammoniumbromid zu und tropft unter guter Kühlung bei 0-1o°C 224 g (2 Mol) 5o%ige Kalilauge zu. Anschließend trennt man die Toluolphase sofort ab, wäscht neutral und rotiert ein. Anschließend wird das

rohe Dien überdestilliert (Siedepunkt 13o-159$^{o}$C/o,2 mm) und anschließend durch eine zweite Destillation gereinigt. Man erhält 67 g 1-(4-Chlorphenyl)-1-chloro-4-methyl-penta-1,3-dien vom Siedepunkt 1oo-1o9$^{o}$C/o,1 mm.

Analog wird aus 2-Methyl-5-oxo-5-phenyl-2(3)-penten (hergestellt nach J.Org.Chem. 25, S. 272) 1-Phenyl-1-chlor-4-methyl-penta-1,3-dien erhalten. Siedepunkt 92-96$^{o}$C/o,o8 mm.

Beispiel zu Verfahren 4:

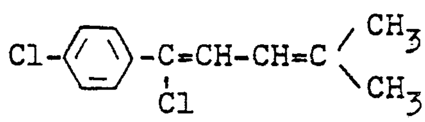

4,6 g (o,2 Mol) Natrium werden portionsweise in 1oo ml trockenem Äthanol gelöst. Wenn sich alles Natrium aufgelöst hat, werden 1oo ml wasserfreies Tetrahydrofuran zugesetzt und bei 0$^{o}$C unter Rühren 59,4 g (o,2 Mol) 4-Chloro-α-chloro-benzyl-phosphonsäurediäthylester, gelöst in 5o ml wasserfreiem Tetrahydrofuran, zugetropft. Nachdem noch 1 Stunde bei 0-1o$^{o}$C nachgerührt wurde, werden 16,8 g (o,2 Mol) ß,ß-Dimethylacrolein, gelöst in 3o ml wasserfreiem Tetrahydrofuran, unter Rühren zugetropft. Anschließend wird noch 12 Stunden bei Raumtemperatur nachgerührt. Die Reaktionsmischung wird dann mit 6oo ml Wasser versetzt und zweimal mit je 3oo ml Methylenchlorid extrahiert. Die organische Phase wird abgetrennt, über Magnesiumsulfat getrocknet,



BAD ORIGINAL

das Lösungsmittel im Wasserstrahlvakuum abdestilliert und der Rückstand im Vakuum destilliert. Man erhält 25,2 g 1-(4-Chloro-phenyl)-1-chloro-4-methyl-penta-1,3-dien (Isomerengemisch) als gelbes, nach einiger Zeit teilweise kristallisierendes Öl, mit dem Siedepunkt 125-134°C / 2 Torr.

Analog erhält man:

1-(Phenyl)-1-chloro-4-methyl-penta-1,3-dien

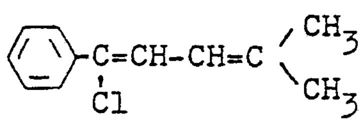

Siedepunkt: 12o-135°C/ 2 Torr

1-(4-Fluoro-phenyl)-1-chloro-4-methyl-penta-1,3-dien

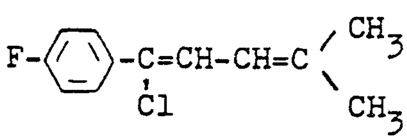

1-(4-Methyl-phenyl)-1-chloro-4-methyl-penta-1,3-dien

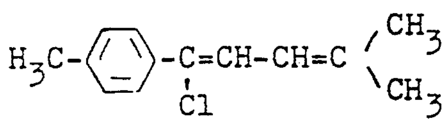

Der als Vorprodukt benötigte α-Hydroxy-benzyl-phosphonsäureester der Formel

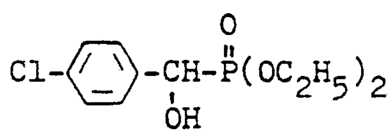

kann wie folgt hergestellt werden:

In ein Gemisch aus 2o,7 g (o,15 Mol) Diäthylphosphit und 1,o9 g (o,olo9 Mol) Triäthylamin werden innerhalb einer Stunde unter Wasserkühlung bei 5o-7o°C 21 g (o,15o Mol) p-Chlorbenzaldehyd eingeleitet. Der Reaktionsansatz wird 1 Stunde bei 7o°C nachgerührt. Nach dem Abkühlen wird der Ansatz mit 4o g Toluol aufgenommen und mehrfach mit verdünnter Salzsäure und kaltem Wasser nachgewaschen. Die organische Schicht wird abgetrennt und im Vakuum vom Lösungsmittel befreit. Der feste Rückstand schmilzt bei 7o-72°C. Die Ausbeute beträgt 37 g (88,5 % der Theorie) an 4-Chloro-α-hydroxy-benzyl-phosphonsäure-diäthylester.

Analog können erhalten werden:

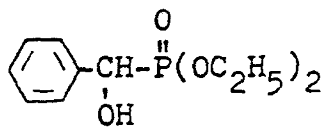

Schmelzpunkt: 75°C

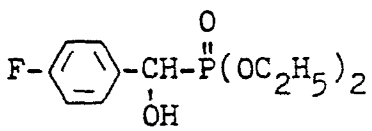

Schmelzpunkt: 26°C

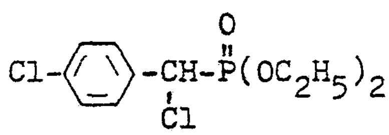

Einem Gemisch aus 2o,2 g (o,o725 Mol) 4-Chloro-α-hydroxy-benzylphosphonsäure-diäthylester, 65 g Dichloromethan und 5,8 g (o,o725 Mol) Pyridin werden bei 2o-4o°C unter leichter Wasserkühlung innerhalb von etwa 1 Stunde 9,2 g (o,o768 Mol) Thionylchlorid hinzugesetzt. Das Reaktionsgemisch wird dann 3 Stunden unter Rückfluß erhitzt und 12 Stunden ohne weitere Wärmeeinwirkung nachgerührt. Das Gemisch wird auf etwa loo g Eiswasser gegossen, die organische Phase abgetrennt und getrocknet. Nach Abdestillieren des Lösungsmittels wird der Rückstand bei 6 Torr und 45°C eingeengt. Man erhält 21 g (97,7% der Theorie) 4-Chloro-α-chloro-benzyl-phosphonsäure-diäthylester als gelbes viskoses Öl mit einer Reinheit von 98,6% (Gaschromatogramm) und einem Brechungsindex von $n_D^{24}$: 1,525o.

Analog können erhalten werden:

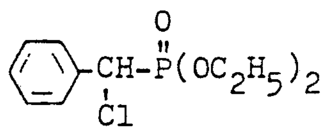

Brechungsindex
$n_D^{23}$: 1,5117

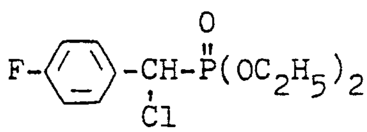

Beispiel zu Verfahren 5:

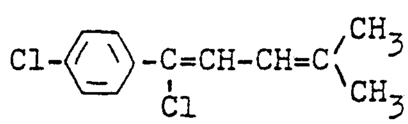

Zu einer Suspension von 22,7 g (o,o525 Mol) Triphenylisopropylphosphoniumjodid in loo ml Tetrahydrofuran werden bei 0°C o,o55 Mol n-Butyl-lithium in 3o ml Hexan getropft. Es wird 1 Stunde bei 0°C nachgerührt. Dann wird in einem Guß eine Lösung von lo,1 g (o,o5 Mol) ß-(4-Chloro-phenyl)-ß-chloro-acrolein in loo ml Tetrahydrofuran zugegeben und dann lo Stunden bei 2o°C nachgerührt. Das Reaktionsgemisch wird darauf eingeengt, mit loo ml Wasser versetzt und zweimal mit je 5o ml Äther extrahiert. Die vereinigten Ätherextrakte werden über Natriumsulfat getrocknet und eingeengt. Es verbleibt ein Rückstand, der bei Zugabe von ca. 5o ml Petroläther kristallisiert. Die Kristalle werden abgesaugt und noch einmal aus Petroläther umkristallisiert.

Man erhält 8 g (7o,5% der Theorie) 1-(4-Chloro-phenyl)-1-chloro-4-methyl-penta-1,3-dien in Form von hellgelben Kristallen mit dem Schmelzpunkt 53°C.

Beispiel zu Verfahren 6:

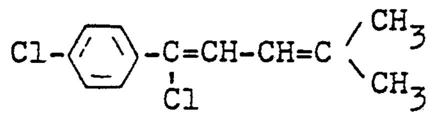

Zu einer Lösung von o,4 Mol Isopropylmagnesiumchlorid in 4o ml Isopropylchlorid (hergestellt nach J.Houben et al.Chem.Ber.69 (1936), 1766) werden bei 2o°C 4o g (o,2 Mol) ß-Chlor-ß-(4-chlorphenyl)-acrolein in 2oo ml Tetrahydrofuran getropft. Es wird 12 Stunden nachgerührt. Dann wird die Reaktionsmischung unter Rühren in eine Mischung von 5oo g Eis und 1oo ml Schwefelsäure gegossen. Es wird zweimal mit je 1oo ml Äther extrahiert. Die vereinigten Ätherextrakte werden über Natriumsulfat getrocknet und bei 5o°C am Rotationsverdampfer eingeengt. Der Rückstand wird in 5o ml Eisessig aufgenommen. Zu dieser Lösung werden 2 ml konzentrierter Schwefelsäure in der Weise getropft, daß die Temperatur 45°C nicht übersteigt. Es wird 4 Stunden bei 2o°C nachgerührt. Dann wird die Reaktionsmischung in Wasser gegeben. Es wird zweimal mit je 1oo ml Äther extrahiert. Die vereinigten Ätherextrakte werden über Natriumsulfat getrocknet, eingeengt und destilliert. Man erhält 23 g 1-(4-Chlorphenyl)-1-chloro-4-methyl-penta-1,3-dien in Form eines gelben Öles, das beim Abkühlen teilweise kristallisiert. Siedepunkt 14o-145°C/4 mm.

Beispiel zu Verfahren 7:

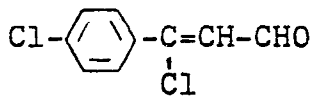

772,5 g (5 Mol) 4-Chloracetophenon werden in 2,5 l Dimethylformamid gelöst, bei 4o-45°C unter leichter Kühlung innerhalb von 2 Stunden tropfenweise mit 157o g Phosphoroxychlorid versetzt, 1 Stunde verrührt, auf 25 l Eiswasser ausgetragen und durch Zutropfen von ca. 2,1 l konzentrierter Natronlauge ein pH-Wert von 5 eingestellt. Die Temperatur wird während der Neutralisation durch Einwerfen von Eis unter 25°C gehalten. Anschließend wird noch 1 Stunde bei pH 5 und bei einer Temperatur unter 25°C nachgerührt. Der helle kristalline Niederschlag wird abgesaugt, gründlich mit Wasser gewaschen und bei 5o°C im Vakuum getrocknet. Ausbeute 74o g (74 % der Theorie); Schmelzpunkt 98°C.

Patentansprüche:

1. Verfahren zur Herstellung von 3-(2-Aryl-vinyl)-2,2-dimethyl-cyclopropan-1-carbonsäureestern der Formel I

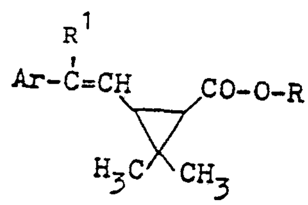

(I)

in welcher

Ar für substituierte oder unsubstituierte carbocyclische oder heterocyclische aromatische Reste steht und

R für Alkyl sowie für Reste der bei Pyrethroiden üblichen Alkohole steht, und

$R^1$ für Wasserstoff oder Halogen steht,

dadurch gekennzeichnet, daß man 1-Aryl-4-methyl-penta-1,3-diene der Formel II

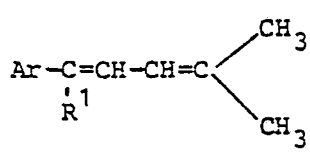

(II)

in welcher
Ar und $R^1$ die oben angegebene Bedeutung hat,

mit Diazoessigsäureestern der Formel III

$$N_2CH\text{-}COO\text{-}R \qquad (III)$$

in welcher
R die oben angegebene Bedeutung hat,

in Gegenwart eines Katalysators bei Temperaturen zwischen 5o und 2oo°C umsetzt.

2. 1-Aryl-4-methyl-penta-1,3-diene der Formel (II)

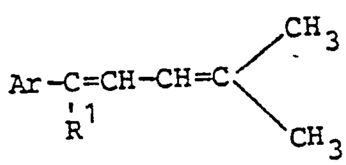

(II)

worin Ar und $R^1$ die oben angegebene Bedeutung haben.

3. Verfahren zur Herstellung von 1-Halogen-1-aryl-4-methyl-penta-1,3-dienen der Formel II (oben), worin Ar die oben angegebene Bedeutung hat, und $R^1$ für Halogen steht, das dadurch gekennzeichnet ist, daß man 3-Methyl-buten-2-yl-arylketone der Formel IVa

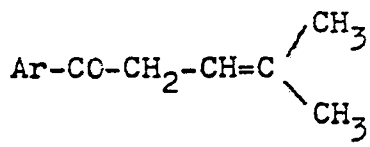

(IV a)

und/oder die dazu isomeren 3-Methyl-buten-1-yl-aryl-ketone der Formel IV b

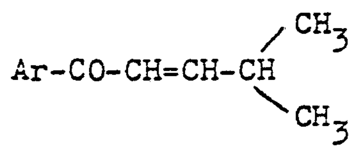

(IV b)

worin Ar die oben angegebene Bedeutung hat,

mit Phosphorpentahalogenid, gegebenenfalls unter Verwendung eines inerten Verdünnungsmittels, bei Temperaturen zwischen -4o°C und +3o°C umsetzt und anschließend das Reaktionsgemisch im gleichen Temperaturbereich mit einem Säurebindemittel, gegebenenfalls in Gegenwart eines Katalysators, behandelt.

4. Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man Arylmethan-phosphonsäureester der Formel V

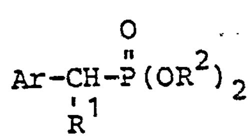

(V)

in welcher

Ar und $R^1$ die oben angegebene Bedeutung hat und

$R^2$ für Alkyl oder Phenyl steht oder worin die beiden Reste $R^2$ zusammen für geradkettiges oder verzweigtes Alkandiyl stehen,

mit Dimethylacrolein der Formel VI

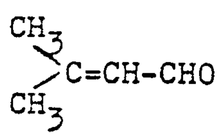 (VI)

in Gegenwart einer Base und gegebenenfalls unter Verwendung eines Verdünnungsmittels bei Temperaturen zwischen -7o und +15o°C umsetzt.

5. Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man ß-Aryl-acroleine der Formel VII

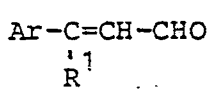 (VII)

in welcher
Ar und $R^1$ die oben angegebene Bedeutung haben,

mit Triphenyl-isopropyl-phosphoran der Formel VIII

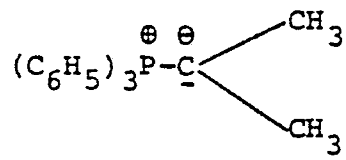

(VIII)

gegebenenfalls unter Verwendung von Verdünnungsmitteln bei Temperaturen zwischen -70 und +50°C umsetzt.

6. Verfahren zur Herstellung von 1-Aryl-4-methyl-penta-1,3-dienen der Formel (II) (oben), worin Ar und $R^1$ die oben angegebene Bedeutung haben, das dadurch gekennzeichnet ist, daß man ß-Aryl-acroleine der Formel VII (oben), worin Ar und $R^1$ die oben angegebene Bedeutung haben, mit Isopropylmagnesiumchlorid der Formel IX

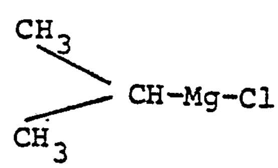

(IX)

gegebenenfalls in Gegenwart von Verdünnungsmitteln bei Temperaturen zwischen -7o und +5o°C umsetzt und anschließend die dabei gebildeten Produkte durch Behandeln mit Säuren bei 0 bis 5o°C dehydratisiert.

7. Verfahren zur Herstellung von ß-Halogen-ß-aryl-acroleinen der Formel VII (oben), worin Ar die oben angegebene Bedeutung hat, und $R^1$ für Halogen steht,dadurch gekennzeichnet, daß man Aryl-methylketone der Formel X

$$Ar-CO-CH_3 \qquad (X)$$

in welcher Ar die oben angegebene Bedeutung hat,

mit Dimethylformamid und Phosphoroxyhalogenid bei Temperaturen zwischen 0 und 100°C ohne Zusatz eines weiteren Verdünnungsmittels umsetzt und die Reaktionsprodukte ohne destillative Aufarbeitung isoliert.

Europäisches Patentamt

# EUROPÄISCHER RECHERCHENBERICHT

Nummer der Anmeldung

EP 80101998.5

| EINSCHLÄGIGE DOKUMENTE | | | KLASSIFIKATION DER ANMELDUNG (Int Cl. 3) |
|---|---|---|---|
| Kategorie | Kennzeichnung des Dokuments mit Angabe, soweit erforderlich, der maßgeblichen Teile | betrifft Anspruch | |
| | FR - A1 - 2 383 914 (IMPERIAL CHEMICAL)<br>+ Patentansprüche 8-11 +<br>-- | 1 | C 07 C 69/753<br>C 07 C 15/44<br>C 07 C 25/24<br>C 07 C 21/24<br>C 07 C 47/24 |
| | DE - A - 2 223 331 (SUMITOMO)<br>+ Patentansprüche 1,2 +<br>-- | 1 | |
| | DE - A1 - 2 706 184 (CIBA-GEIGY)<br>+ Patentansprüche 1,6,7,10 +<br>-- | 1 | |
| D | DE - A1 - 2 738 150 (FMC CORP.)<br>+ Patentanspruch 1 +<br>-- | 1 | RECHERCHIERTE SACHGEBIETE (Int. Cl. 3)<br>C 07 C 69/00<br>C 07 C 15/00<br>C 07 C 43/00<br>C 07 C 47/00<br>C 07 C 25/00<br>C 07 C 21/00 |
| | GB - A - 813 539 (BADISCHE ANILIN)<br>+ Patentanspruch 1 +<br>-- | 2,5 | |
| | DE - A - 2 339 671 (SANDOZ)<br>+ Patentanspruch 1 Abschnitt e +<br>-- | 2,5 | |
| | AT - B - 283 320 (HOECHST...)<br>+ Patentanspruch +<br>-- | 7 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | GB - A - 891 178 (HOECHST...)<br>+ Patentanspruch 1 +<br>-- | 2,4 | |
| P | DE - A1 - 2 848 495 (CIBA-GEIGY) (17-05-1980)<br>+ Patentanspruch 1 +<br>-- | 1 | &: Mitglied der gleichen Patentfamilie, übereinstimmendes Dokument |
| X | Der vorliegende Recherchenbericht wurde für alle Patentansprüche erstellt | | |

| Recherchenort | Abschlußdatum der Recherche | Prüfer |
|---|---|---|
| WIEN | 19-06-1980 | REIF |

Europäisches Patentamt

**EUROPÄISCHER RECHERCHENBERICHT**

Nummer der Anmeldung

EP 80101998.5

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| P | DE - A1 - 2 827 101 (BAYER) (10-01-1980)<br>+ Patentanspruch +<br>& EP-A1-6 205 (09-01-1980)<br>---- | 1 | |
| | | | RECHERCHIERTE SACHGEBIETE (Int. Cl. 3) |